# EUROPEAN PATENT SPECIFICATION

(11) **EP 1 404 389 B1**
(45) Date of publication and mention of the grant of the patent: **12.01.2005**
(21) Application number: 02764656.1
(22) Date of filing: 09.07.2002
(51) Int. Cl.: A61L 27/30, A61L 31/08

(54) **BIOMEDICAL TITANIUM IMPLANTS**
BIOMEDIZINISCHE TITANIMPLANTATE
IMPLANTS DE TITANE BIOMEDICAUX

(30) Priority: 09.07.2001 GB 0116725
(43) Date of publication of application: 07.04.2004
(73) Proprietor: The European Community, represented by the European Commission, 1049 Brussels (BE)
(72) Inventor: STROOSNIJDER, Rien, EC, Joint Research Cntr./Ispra, 21020 Ispra (IT); BILLI, Fabrizio, EC, Joint Research Cntr./Ispra, 21020 Ispra (IT)
(74) Representative: Schmitt, Armand
(86) International application number: PCT/EP2002/007628
(87) International publication number: WO 2003/006080

(56) References cited:
- WO-A-00/72777
- US-A- 4 746 532
- US-A- 5 667 385
- GOBEL M ET AL: "Study of the high-temperature oxidation behaviour of Ti and Ti4Nb with SNMS using tracers" SURFACE AND INTERFACE ANALYSIS, MAY 2000, WILEY, UK, vol. 29, no. 5, pages 321-324, XP008010708 ISSN: 0142-2421
- CODDET C ET AL: "Experimental interpretation of the mechanism of oxidation of the titanium alloy TA6V4 by dry oxygen between 600 degrees C and 1000 degrees C" JOURNAL OF THE LESS-COMMON METALS, JAN. 1977, SWITZERLAND, vol. 51, no. 1, pages 1-12, XP001109198 ISSN: 0022-5088

## Description

The present invention relates to the field of biomedical implants and, in particular, to a process for forming an oxide layer on the surface of a titanium or titanium-based biomedical implant.

A requirement in the use of endosseous implants, for example endosseous dental implants, is to achieve osseo-integration and/or bio-integration of the implant with the bone.

A wide variety of materials have been used for biomedical implants, but only a few fulfill the osseo-integration requirement to a sufficient level. Titanium and titanium-based alloys are the preferred materials used for such implants. The nature of the native surface oxide of the titanium appears to be the underlying factor for good osseo-integration. The surface oxide appears to promote the deposition of biological molecules and to limit the dissolution of the bulk elements. It is believed that this release determines the biological response to the dental implant.

Although osseo-integrated titanium implants have a long and successful record in treating edentulous patients with normal bone stock, the results obtained from clinical intervention are not as good when bone quantity and quality is inferior. In such cases, achieving an improved level of osseo-integration is an important factor in producing a successful implant. Additionally, improved osseo-integration may reduce the time required to achieve an adequate load bearing capability which, at present, may be up to 6 months for the upper jaw. The critical role of surface condition on the clinical performance of titanium based materials has meant that efforts to improve their behaviour have concentrated not only on the alloy composition but also on the use of surface treatments.

Besides endosseous dental implants, a wide range of other biomedical implants have similar requirements for good osseo-integration.

Different surface treatments have been applied to improve the adhesion of cells to biomedical implants, including grit blasting of the surface with particles of an oxide of titanium and applying ceramic coatings. Application of porous coatings, such as, for example, a 50-100 µm thick hydroxyapatite (HA) type coating by plasma spraying has become widespread since the early 1980s. However, a number of clinical questions regarding their efficacy have been raised, including the mechanical weakness of the coating/substrate interface and the difficulty of producing a uniform layer on complex shaped implants. Another difficulty concerning plasma spraying is that it does not allow the deposition of coatings thinner than about 40 µm. The long-term behaviour of these ceramic coatings is jeopardized due to continued (and improperly controlled) dissolution of the coating. The crystallinity of the coating is a major factor in determining dissolution rates with amorphous coatings being more prone to biodegradation than crystalline ones. There is also concern over the possible increase in corrosion rate, both because of increased surface area and crevice corrosion effects. Other ceramic coatings have been produced by other methods including electrochemical treatments, but up till now these have not shown great success. Heat treating in air has also been reported as a method of achieving a reduction in ion release from a Ti-based alloy into physiological solutions. Several factors might affect ion release from the oxidized surface of the metal, such as increased oxide thickness (typically ≥ ≈ 4 nm compared with ≈ 2.5 nm for the native oxide) and a modified oxide structure.

US-A-4,746,532 discloses a method for producing endosseous implants by spraying a ceramic material onto the surface of a metallic titanium core material which is previously subjected to a surface oxidation treatment by heat treatment in air. US-A-4,746,532 does not disclose any effect related to the humidity of the air used during the oxidation process.

Accordingly, the present invention provides a process for the production of a biomedical implant having an oxide of titanium on a surface thereof, the process comprising:
(a) providing a biomedical implant, at least a surface of which comprises titanium or an alloy thereof;
(b) exposing said surface of the biomedical implant to an oxygen-containing atmosphere having a dew point of ≤ 0°C; and
(c) heating said surface of the biomedical implant in said atmosphere whereby an oxide of titanium is formed on at least said surface.

The dew point of the oxygen-containing atmosphere provides an indication of the humidity of the atmosphere. The atmosphere will generally have a dew point of ≤ 0°C, preferably from -60°C to 0°C, more preferably from -50°C to -5°C, more preferably from -40°C to -10°C, still more preferably from -30°C to -15°C.

The process according to the present invention has been found to result in an improvement in the osseo-integration of the biomedical implant compared with prior art titanium and titanium-based implants. In particular, the surface morphology of the oxide scale formed in atmospheres having a dew point of from ≤ 0°C appears to be more homogeneous than that formed upon oxidation in a more humid environment, i.e. an environment having a higher dew point. Thus, using an environment having the recited dew point, a less defective and more adherent oxide scale may be formed, which is characterised by a more uniform outer surface.

The dew point of the oxygen-containing atmosphere may be maintained at a substantially constant level during the heating step or, alternatively, may be varied during the heating step. The latter approach may be used to produce variations in the chemistry and/or microstructure of the oxide layer with depth. Accordingly, it is possible to combine two or more thermal treatments in environments having different levels of humidity in order to obtain a more graded oxide structure.

Heating is typically carried out at a temperature in the range of from 300 to 900°C, more typically from 700 to 900°C, and will generally be performed in a suitable furnace provided with a source of an oxidising gas and an inlet and outlet. Following the heating step the implant may be allowed to cool (eg furnace cooling) or actively cooled by flowing a cooling fluid over the implant. Additional surface treatment steps may then be carried out. The implant will generally be sterilised prior to use.

The surface of the biomedical implant may be maintained at a substantially constant temperature during the heating step. Alternatively, the temperature may be varied during the heating step. Again, this latter approach may be used to produce variations in the chemistry and/or microstructure of the oxide layer with depth. Thermal cycling may be carried out during the oxidation treatment in order to improve adherence of the oxide scale. It will be appreciated that non-isothermal treatments or thermal cycling may be carried out while keeping the humidity of the oxidising atmosphere at a substantially constant level. Alternatively, both the temperature and the humidity of the oxidising atmosphere may be varied.

Heating is typically carried out at a heating rate of up to 100°C/min, more typically from 10 to 70°C/min, still more typically from 30 to 50°C/min.

Heating may be continued for any suitable length of time to obtain the desired oxide morphology and/or thickness. For example, heating may typically be carried out for up to 200 hours, or for up to 150 hours, or for up to 100 hours, or for up to 50 hours, or for up to 10 hours, or for up to 1 hour.

The oxidising atmosphere may comprise or consist of oxygen gas and/or a molecular gas or a vapour that contains oxygen. An example of a suitable atmosphere is air or a gas mixture comprising oxygen and nitrogen. The desired dew point of the atmosphere, which is a measure of its humidity, may be achieved by flowing a proportion or all of one or more of the gases which are comprised in the atmosphere over water. The relative humidity of the atmosphere is preferably less than 10% at 22°C, more preferably less than 5%, still more preferably less than 1%. The water partial pressures is preferably less than 500 Pa, more preferably less than 200 Pa.

The surface of the biomedical implant may comprise commercially pure Ti (cp Ti, Grades 1, 2, 3 or 4)) or the alloy Ti6A14V. Part or all of the biomedical implant may be formed from titanium or an alloy thereof. As will be appreciated, the process according to the present invention may be performed on part or all of the implant.

The term implant as used herein is meant to encompass all types of biomedical and dental implants, including component parts and portions thereof.

The oxide of titanium formed on the surface of the biomedical implant will typically comprise one or more of TiO₂, TiO and Ti₂O₃.

The biomedical implant is advantageously an endosseous implant, preferably an endosseous dental implant, including component parts and portions thereof.

It will be appreciated that additional surface modifications to the biomedical implant may be performed before or after the oxidation treatment. Examples include mechanical treatments of' the surface (eg shot blasting), chemical treatments (eg etching), and physical treatments (eg ion implantation).

The process according to the present invention may, if desired, further comprise a sterilisation step.

The present invention also provides a method of forming an oxide layer on the surface of a biomedical implant formed from titanium or an alloy thereof, the method comprising:
(i) exposing the biomedical implant to an oxygen-containing atmosphere having a dew point of ≤ 0°C; and
(ii) heating at least a surface of the biomedical implant in said atmosphere to form an oxide of titanium thereon.

The present invention also provides a method of improving the osseo-integration of a biomedical implant formed from titanium or an alloy thereof, the method comprising:
(i) exposing the biomedical implant to an oxygen-containing atmosphere having a dew point of ≤ 0°C; and
(ii) heating at least a surface of the biomedical implant in said atmosphere to form an oxide of titanium thereon.

For the avoidance of doubt, all of the features described in relation to the process according to the present invention are equally applicable, either singularly or in combination to the methods described herein.

The present invention also provides a biomedical implant obtainable or whenever produced by a process or method as herein described.

The present invention will now be described further, by way of example, with reference to the following drawings in which:

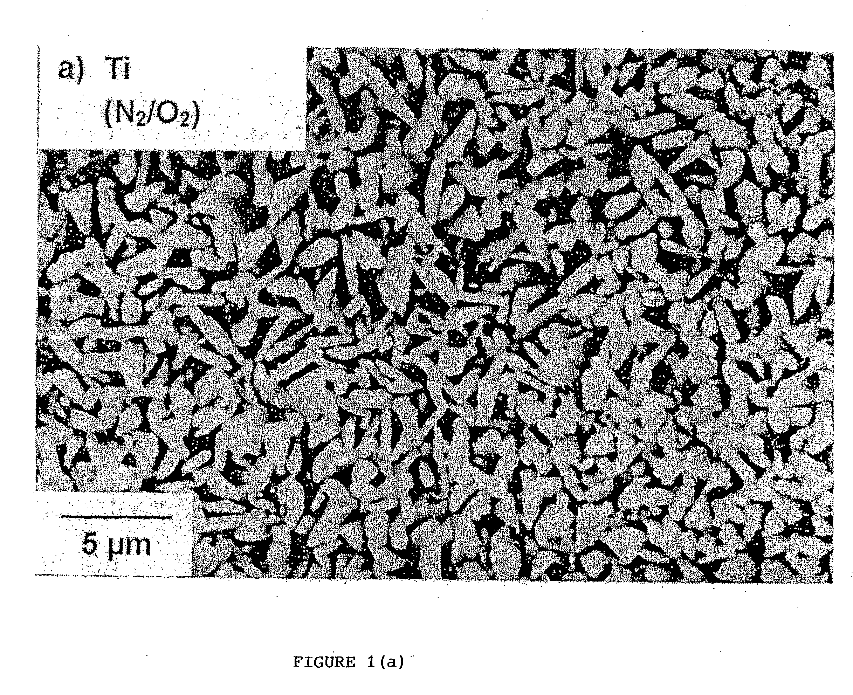

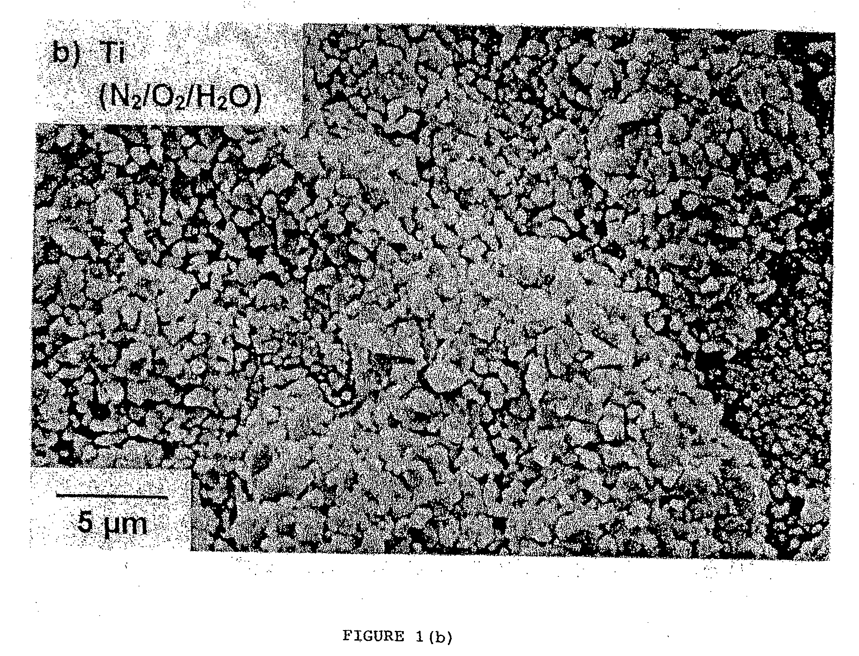

Figure 1(a) is a SEM micrograph showing the surface morphology of titanium exposed at 800°C for 150 hours in dry air (dew point less than -40°C); and
Figure 1(b) is a SEM micrograph showing the surface morphology of titanium exposed at 800°C for 150 hours in humid air (dew point approximately 8° C).

It will be seen that titanium sample shown in the micrograph in Figure 1(a), which is in accordance with the present invention, has a much more homogeneous structure than that shown in Figure 1(b), which is not in accordance with the present invention.

In the present invention, a surface modification of a Ti or Ti-based surgical or biomedical implant is effected by a thermal treatment in an oxidising environment such as, for example, air. The humidity of the atmosphere is controlled at a level lower than that present in normal laboratory air. In other words the air is dried. It is believed that the presence of water increases the oxidation rate.

The process and methods according to the present invention provide a number of advantages over the prior art. First, the present invention results in an improvement in the osseo-integration of the biomedical implant. Second, the adherence of the oxide scale to the underlying base material is also improved. Third, by its very nature the present invention enables complex-shaped implants to be substantially uniformly surface-treated. Fourth, it has been found that process and methods according to the present invention also result in a cleaning action, by which contaminants may be removed.

## Claims

1. A process for the production of a biomedical implant having an oxide of titanium on a surface thereof, the process comprising:
(a) providing a biomedical implant, at least a surface of which comprises titanium or an alloy thereof;
(b) exposing said surface of the biomedical implant to an oxygen-containing atmosphere having a dew point of ≤ 0°C;
(c) heating said surface of the biomedical implant in said atmosphere whereby an oxide of titanium is formed on at least said surface.

2. A process as claimed in claim 1, wherein said oxygen-containing atmosphere has a dew point of from -60°C to 0°C, preferably from -50°C to -5°C, more preferably from -40°C to -10°C, still more preferably from -30°C to -15°C.

3. A process as claimed in claim 1 or claim 2, wherein the dew point of the oxygen-containing atmosphere is maintained at substantially a constant level during the heating step.

4. A process as claimed in claim 1 or claim 2, wherein the dew point of the oxygen-containing atmosphere is varied during the heating step.

5. A process as claimed in any one of the preceding claims, wherein heating of said surface of the biomedical implant is carried out at a temperature in the range of from 300 to 900°C, preferably from 700 to 900°C.

6. A process as claimed in any one of the preceding claims, wherein said surface of the biomedical implant is maintained at a substantially constant temperature during the heating step.

7. A process as claimed in any one of claims 1 to 5, wherein the temperature of said surface of the biomedical implant is varied during the heating step.

8. A process as claimed in any one of the preceding claims, wherein heating of said surface of the biomedical implant is carried out at a heating rate of up to 100°C/min, preferably from 10 to 70°C/min, more preferably from 30 to 50°C/min.

9. A process as claimed in any one of the preceding claims, wherein said oxygen-containing atmosphere comprises or consists of oxygen gas and/or a molecular gas or vapour that contains oxygen.

10. A process as claimed in any one of the preceding claims, wherein said surface of the biomedical implant comprises commercially pure Ti (cp Ti, Grades 1, 2, 3 or 4)) or the alloy Ti6Al4V.

11. A process as claimed in any one of the preceding claims, wherein the oxide of titanium formed on at least said surface of the biomedical implant comprises one or more of TiO₂, TiO and Ti₂O₃.

12. A process as claimed in any one of the preceding claims, wherein the biomedical implant is an endosseous implant, preferably an endosseous dental implant.

13. A method of forming an oxide layer on the surface of a biomedical implant formed from titanium or an alloy thereof, the method comprising:
(i) exposing the biomedical implant to an oxygen-containing atmosphere having a dew point of ≤ 0°C; and
(ii) heating at least a surface of the biomedical implant in said atmosphere to form an oxide of titanium thereon.

14. A method of improving the osseo-integration and/or bio-integration of a biomedical implant formed from titanium or an alloy thereof, the method comprising:
(i) exposing the biomedical implant to an oxygen-containing atmosphere having a dew point of ≤ 0°C; and
(ii) heating at least a surface of the biomedical implant in said atmosphere to form an oxide of titanium thereon.

15. A biomedical implant obtainable by a process as claimed in any one of claims 1 to 12 or a method as claimed in claim 13 or claim 14.

## Patentansprüche

1. Verfahren zur Herstellung eines biomedizinischen Implantats mit einem Oxid von Titan auf einer Oberfläche davon, wobei das Verfahren umfasst:
a) Bereitstellung eines biomedizinischen Implantats, von dem zumindest eine Oberfläche Titan oder eine Legierung davon umfasst;
b). Exposition der Oberfläche des biomedizinischen Implantats in einer sauerstoffhaltigen Atmosphäre mit einem Taupunkt von ≤ 0°C; und
c) Erhitzung der Oberfläche des biomedizinischen Implantats in der Atmosphäre, wodurch ein Oxid von Titan zumindest auf der Oberfläche gebildet wird.

2. Verfahren nach Anspruch 1, wobei die sauerstoffhaltige Atmosphäre einen Taupunkt von -60°C bis 0°C, vorzugsweise von -50°C bis -5°C, bevorzugter von -40°C bis -10°C, noch bevorzugter von -30°C bis -15°C aufweist.

3. Verfahren nach Anspruch 1 oder 2, wobei der Taupunkt der sauerstoffhaltigen Atmosphäre während des Erhitzungsschritts bei einem im wesentlichen konstanten Wert aufrechterhalten wird.

4. Verfahren nach Anspruch 1 oder 2, wobei der Taupunkt der sauerstoffhaltigen Atmosphäre während des Erhitzungsschritts verändert wird.

5. Verfahren nach irgendeinem der vorangehenden Ansprüche, wobei die Erhitzung der Oberfläche des biomedizinischen Implantats bei einer Temperatur im Bereich von 300 bis 900°C, vorzugsweise von 700 bis 900°C durchgeführt wird.

6. Verfahren nach irgendeinem der vorangehenden Ansprüche, wobei die Oberfläche des medizinischen Implantats während des Erhitzungsschritts bei einer im wesentlichen konstanten Temperatur gehalten wird.

7. Verfahren nach irgendeinem der Ansprüche 1 bis 5, wobei die Temperatur der Oberfläche des biomedizinischen Implantats während des Erhitzungsschritts verändert wird.

8. Verfahren nach irgendeinem der vorangehenden Ansprüche, wobei die Erhitzung der Oberfläche des biomedizinischen Implantats bei einer Aufheizgeschwindigkeit von bis zu 100°C/min, vorzugsweise von 10 bis 70°C/min, bevorzugter von 30 bis 50°C/min durchgeführt wird.

9. Verfahren nach irgendeinem der vorangehenden Ansprüche, wobei die sauerstoffhaltige Atmosphäre Sauerstoffgas und/oder ein sauerstoffhaltiges molekulares Gas oder einen sauerstoffhaltigen Dampf umfasst oder daraus besteht.

10. Verfahren nach irgendeinem der vorangehenden Ansprüche, wobei die Oberfläche des biomedizinischen Implantats technisch reines Ti (cp Ti, Sorten 1, 2, 3 oder 4) oder die Legierung Ti6Al4V umfasst.

11. Verfahren nach irgendeinem der vorangehenden Ansprüche, wobei das zumindest auf der Oberfläche des biomedizinischen Implantats gebildete Oxid von Titan eines oder mehrere von TiO₂, TiO und Ti₂O₃ umfasst.

12. Verfahren nach irgendeinem der vorangehenden Ansprüche, wobei das biomedizinische Implantat ein endosteales Implantat, vorzugsweise ein endosteales Zahnimplantat ist.

13. Methode zur Bildung einer Oxidschicht auf der Oberfläche eines aus Titan oder einer Legierung davon gebildeten biomedizinischen Implantats, wobei die Methode umfasst:
i) Exposition des biomedizinischen Implantats in einer sauerstoffhaltigen Atmosphäre mit einem Taupunkt von ≤ 0°C; und
ii) Erhitzung zumindest einer Oberfläche des biomedizinischen Implantats in der Atmosphäre, um darauf ein Oxid von Titan zu bilden.

14. Methode zur Verbesserung der Osseointegration und/oder Biointegration eines aus Titan oder einer Legierung davon gebildeten biomedizinischen Implantats, wobei die Methode umfasst:
i) Exposition des biomedizinischen Implantats in einer sauerstoffhaltigen Atmosphäre mit einem Taupunkt von ≤ 0°C; und
ii) Erhitzung zumindest einer Oberfläche des biomedizinischen Implantats in der Atmosphäre, um darauf ein Oxid von Titan zu bilden.

15. Biomedizinisches Implantat, erhältlich durch ein Verfahren nach irgendeinem der Ansprüche 1 bis 12 oder eine Methode nach Anspruch 13 oder 14.

## Revendications

1. Procédé pour la fabrication d'un implant biomédical portant un oxyde de titane en surface, ledit procédé comprenant :
(a) la mise en oeuvre d'un implant biomédical, dont au moins une surface comprend du titane ou un alliage de celui-ci ;
(b) l'exposition de ladite surface de l'implant biomédical à une atmosphère contenant de l'oxygène ayant un point de rosée ≤ 0° C ;
(c) le chauffage de ladite surface de l'implant biomédical dans ladite atmosphère, permettant ainsi la formation d'un oxyde de titane sur au moins ladite surface.

2. Procédé selon la revendication 1, dans lequel ladite atmosphère contenant de l'oxygène a un point de rosée situé entre -60° C et 0° C, de préférence entre -50° C et -5° C, de préférence encore entre -40° C et -10° C, avec une nette préférence entre -30° C et -15° C.

3. Procédé selon la revendication 1 ou 2, dans lequel le point de rosée de l'atmosphère contenant de l'oxygène est maintenu à un niveau pratiquement constant pendant l'étape de chauffage.

4. Procédé selon la revendication 1 ou 2, dans lequel on fait varier le point de rosée de l'atmosphère contenant de l'oxygène pendant l'étape de chauffage.

5. Procédé selon l'une quelconque des revendications précédentes, dans lequel le chauffage de ladite surface de l'implant biomédical est effectué à une température située dans la plage de 300° à 900° C, de préférence entre 700° et 900° C.

6. Procédé selon l'une quelconque des revendications précédentes, dans lequel ladite surface de l'implant biomédical est maintenue à une température pratiquement constante pendant l'étape de chauffage.

7. Procédé selon l'une quelconque des revendications 1 à 5, dans lequel on fait varier la température de ladite surface de l'implant biomédical pendant l'étape de chauffage.

8. Procédé selon l'une quelconque des revendications précédentes, dans lequel le chauffage de ladite surface de l'implant biomédical est effectué à une allure de chauffage allant jusqu'à 100°C/min, de préférence de 10° à 70°C/min, de préférence encore de 30° à 50°C/min.

9. Procédé selon l'une quelconque des revendications précédentes, dans lequel ladite atmosphère contenant de l'oxygène comprend ou est constituée d'oxygène et/ou d'un gaz ou d'une vapeur moléculaire contenant de l'oxygène.

10. Procédé selon l'une quelconque des revendications précédentes, dans lequel ladite surface de l'implant biomédical comprend du titane (Ti) industriellement pur (cp Ti, qualités 1, 2, 3 ou 4) ou l'alliage Ti6Al4V.

11. Procédé selon l'une quelconque des revendications précédentes, dans lequel l'oxyde de titane formé sur au moins ladite surface de l'implant biomédical comprend un ou plusieurs composés parmi TiO₂, TiO et Ti₂O₃.

12. Procédé selon l'une quelconque des revendications précédentes, dans lequel l'implant biomédical est un implant endo-osseux, de préférence un implant dentaire endo-osseux.

13. Méthode de formation d'une couche d'oxyde sur la surface d'un implant biomédical formé de titane ou d'un alliage de celui-ci, ladite méthode comprenant :
(i) l'exposition de l'implant biomédical à une atmosphère contenant de l'oxygène ayant un point de rosée ≤ 0° C ; et
(ii) le chauffage d'au moins une surface de l'implant biomédical dans ladite atmosphère pour former dessus un oxyde de titane.

14. Méthode d'amélioration de l'intégration osseuse et/ou de la bio-intégration d'un implant biomédical formé de titane ou d'un alliage de celui-ci, ladite méthode comprenant :
(i) l'exposition de l'implant biomédical à une atmosphère contenant de l'oxygène ayant un point de rosée ≤ 0° C ; et
(ii) le chauffage d'au moins une surface de l'implant biomédical dans ladite atmosphère pour former dessus un oxyde de titane.

15. Implant biomédical susceptible d'être obtenu par un procédé selon l'une quelconque des revendications 1 à 12 ou une méthode selon la revendication 13 ou 14.
